# EUROPÄISCHE PATENTSCHRIFT

(11) **EP 2 223 111 B1**
(45) Veröffentlichungstag und Bekanntmachung des Hinweises auf die Patenterteilung: **26.07.2017**
(21) Anmeldenummer: 08843381.8
(22) Anmeldetag: 29.10.2008
(51) Int. Cl.: G01N 33/569, G01N 21/64

(54) **ELISPOT-VERFAHREN MIT ZWEI FILTERSYSTEMEN**
ELISPOT METHOD HAVING TWO FILTER SYSTEMS
PROCÉDÉ ELISPOT FAISANT APPEL À DEUX SYSTÈMES DE FILTRATION

(30) Priorität: 29.10.2007 DE 102007052517
(43) Veröffentlichungstag der Anmeldung: 01.09.2010
(73) Patentinhaber: Autoimmun Diagnostika GmbH, 72479 Strassberg (DE)
(72) Erfinder: SCHÖLLHORN, Volkmar, 72458 Albstadt (DE)
(74) Vertreter: Patentanwälte Ruff, Wilhelm, Beier, Dauster & Partner mbB
(86) Internationale Anmeldenummer: PCT/EP2008/009112
(87) Internationale Veröffentlichungsnummer: WO 2009/056282

(56) Entgegenhaltungen:
- EP-A- 0 957 359
- WO-A-02/08754
- WO-A-90/04182
- WO-A-98/23960
- WO-A-2005/106482
- WO-A2-2007/008583
- US-A1- 2003 215 791
- SHIRAI A ET AL: "Individual cells simultaneously produce both IL-4 and IL-6 in vivo" CYTOKINE, ACADEMIC PRESS LTD, PHILADELPHIA, PA, US, Bd. 6, Nr. 3, 1. Mai 1994 (1994-05-01), Seiten 329-336, XP023271493 ISSN: 1043-4666 [gefunden am 1994-05-01]
- D. C. Douek ET AL: "T-cell apoptosis and differential human leucocyte antigen class II expression in human thymus", IMMUNOLOGY., vol. 99, no. 2, 1 February 2000 (2000-02-01), pages 249-256, XP055210195, GB ISSN: 0019-2805, DOI: 10.1046/j.1365-2567.2000.00940.x
- Anna Smallcombe: "Multicolor Imaging: The Important Question of Co-Localization", Biotechniques, vol. 30, no. 6, 1 January 2001 (2001-01-01), pages 1240-1246, XP055210194, US ISSN: 0736-6205
- NILSSEN D E ET AL: "B-cell activation in duodenal mucosa after oral cholera vaccination in IgA deficient subjects with or without IgG subclass deficiency", SCANDINAVIAN JOURNAL OF IMMUNOLOGY, BLACKWELL SCIENCE PUBL., OXFORD, GB, vol. 38, no. 2, 1 January 1993 (1993-01-01), pages 201-208, XP002510247, ISSN: 0300-9475, DOI: 10.1111/J.1365-3083.1993.TB01714.X

## Beschreibung

Die vorliegende Erfindung betrifft ein ELISPOT-Verfahren zur in vitro-Diagnose und/oder in vitro-Therapieüberwachung von Tuberkulose oder Borreliose.

Der sogenannte "Enzyme-linked immunospot assay" (ELISPOT) wird unter anderem zur Messung der antigenspezifischen Aktivität von Blutzellen verwendet. Der Test wird üblicherweise in Mikrotiterplatten mit 96 Kavitäten durchgeführt, wobei jede Kavität am Boden eine Membran trägt, auf der sich bei positivem Ausfall des Tests eine lokale Farbreaktion um einzelne aktivierte Zellen ausbildet. Die Auswertung geschieht durch einfaches Auszählen der Punkte oder unter Einsatz von digitalen Bildverarbeitungssystemen, wobei neben der Anzahl der Punkte auch die Größe und Intensität der Punkte quantifiziert werden kann. Die Farbreaktion beruht auf dem Nachweis von lokal gebildeten Botenstoffen, die über Membran gekoppelte primäre Antikörper gebunden werden. Diese lokal gebundenen Botenstoffe werden dann durch die Bindung eines zweiten, gegen den gleichen Botenstoff gerichteten Antikörpers, der direkt oder indirekt mit einer Farbreaktion gekoppelt wird, visualisiert.

Bisher wurde bei der Aktivitätsmessung von Zellen ausschließlich die Anzahl der reaktiven Zellen gemessen, um daraus Rückschlüsse auf ihre Aktivität ziehen zu können. Diesbezüglich wurde jedoch erkannt, dass es nicht so sehr bzw. nicht nur auf die Anzahl der reagierenden Zellen, sondern vor allem auch auf die Qualität der Reaktion der einzelnen Zellen ankommt. So können einzelne Zellen sehr unterschiedlich auf das gleiche Antigen reagieren, was sich in der Quantität der abgegebenen Botenstoffe und damit in der Quantität der im vorherigen Abschnitt erwähnten Farbreaktion jeder einzelnen Zelle manifestiert. In der EP 0 957 359 A2 wird ein ELISPOT-Verfahren beschrieben, bei welchem nicht nur die Anzahl der reaktiven Zellen ermittelt wird, sondern auch die Intensität der Gesamtreaktion. Dies erlaubt wesentlich weitergehende Schlüsse auf die Aktivität der Zellen.

Shirai et al. (Cytokine 1994, Vol. 6, No. 3: 329 - 336) untersucht die antigenstimulierte Freisetzung von zwei Zytokinen durch Maus-T-Zelllinien mit Hilfe eines für jedes Zytokin getrennt durchgeführten ELISPOT-Verfahrens, wobei die Detektion auf einer kolorimetrischen Reaktion beruht.

Aus der WO 90/04182 A1 ist ein ELISPOT-basiertes Verfahren zur gleichzeitigen Detektion sezernierter Antikörper oder Antigene bekannt, wobei die Detektion ebenfalls auf einer kolorimetrischen Reaktion beru ht.

Die WO 98/23960 A1 betrifft ein ELISPOT-basiertes Verfahren zur Detektion eines von T-Zellen sezernierten Zytokins, wobei die T-Zellen zuvor mit einem Antigen in vivo sensibilisiert wurden.

Die WO 02/08754 A1 beschreibt ein Dualcolor-ELISPOT-Verfahren zur Detektion zweier Analyte, wobei das Verfahren in einer Flusszelle durchgeführt wird.

Aus der WO 2005/106482 A1 ist ein ELISPOT-Verfahren zur Detektion mehrerer Zytokine bekannt, wobei die einzelnen Zytokine räumlich getrennt voneinander mit Hilfe immobilisierter Fängerantikörper detektiert werden.

Ein Nachweisverfahren auf Zytokin-ausscheidende Zellen ist aus der US 2003/0215791 A1 bekannt.

Ein grundsätzliches Problem des ELISPOT-Verfahrens besteht jedoch hinsichtlich der gezielten Erfassung von Zellen, die in Gegenwart eines Antigens zwei oder mehrere verschiedene Botenstoffe ausscheiden. Ein weiteres Problem besteht darin, diese Zellen von anderen Zellen, die lediglich einen Botenstoff ausscheiden, getrennt zu erfassen.

Wie bereits einleitend erwähnt, beruht die Messung von Zellen im Rahmen des ELISPOT-Verfahrens in der Regel auf Farbreaktionen, die auf Chemolumineszenz- oder Fluoreszenz-Eigenschaften entsprechender Farbstoffe zurückgehen. Die zur Chemolumineszenz- bzw. Fluoreszenz-Messung üblicherweise verwendeten Filtersysteme beruhen gewöhnlich auf einem einzigen Filtersatz, der bezüglich der Anregung und der Emission eines Farbstoffes nur für Licht eines bestimmten Wellenlängenbereiches durchlässig ist. Zwar kommen mittlerweile auch sogenannte Dualfilter zum Einsatz. Nachteilig hierbei ist jedoch, dass diese Filter für einen verhältnismäßig breiten Spektralbereich durchlässig sind. Da Farbstoffe allgemein auch außerhalb ihres "idealen" Spektralbereiches Licht absorbieren und emittieren können, würde der Einsatz von Dualfiltern bei der Messung von Zellen, die zwei oder mehrere verschiedene Botenstoffe ausscheiden, zu einer erheblichen Einschränkung der Messgenauigkeit führen. Verstärkt wird dieses Problem dadurch, dass die Botenstoffe gewöhnlich in unterschiedlichen Mengen von besagten Zellen ausgeschieden werden.

Die Erfindung stellt sich daher die Aufgabe, ein ELISPOT-Verfahren bereitzustellen, welches aus dem Stand der Technik bekannte Nachteile vermeidet und insbesondere für eine sensitive Messung von Zellen geeignet ist, welche zwei oder mehrere verschiedene Botenstoffe ausscheiden.

Diese Aufgabe wird erfindungsgemäß gelöst durch ein Verfahren mit den Merkmalen des unabhängigen Anspruchs 1. Bevorzugte Ausführungsformen des Verfahrens sind Gegenstand der abhängigen Ansprüche 2 bis 9. Ein weiterer Aspekt der Erfindung betrifft die Verwendung des Verfahrens gemäß den unabhängigen Ansprüchen 10 und 11. Der Wortlaut sämtlicher Ansprüche wird hiermit durch Bezugnahme zum Inhalt dieser Beschreibung gemacht.

Bei dem erfindungsgemäßen Verfahren handelt es sich um ein ELISPOT-Verfahren ("Enzyme-linked immunospot assay"), insbesondere zur in vitro-Diagnose und/oder in vitro-Therapieüberwachung von Infektionen und/oder Infektionserkrankungen, wobei eurkaryotische Zellen, vorzugsweise auf einer Untersuchungsfläche bzw. einem Träger, mit einem Antigen inkubiert werden und die Anzahl an immunkompetenten Zellen gemessen wird, die zumindest zwei verschiedene Zytokine als Reaktion auf das Antigen ausscheiden, wobei zur Messung der immunkompetenten Zellen diese zumindest mit Hilfe von zwei verschiedenen Farbstoffen und zumindest zwei verschiedenen Filtersätzen visualisiert werden, wobei die Filtersätze Schmalbandfilter aufweisen, wie es in den Ansprüchen definiert ist. Durch die Erfindung wird ein ELISPOT-Verfahren bereitgestellt, welches eine selektive Erfassung von Immunzellen, die zwei oder mehrere verschiedene Zytokine ausscheiden, gegenüber anderen Immunzellen, die lediglich einen Zytokintyp ausscheiden, erlaubt. Die selektive Messung besagter Immunzellen beruht erfindungsgemäß auf der Verwendung von verschiedenen Filtersätzen mit Schmalbandfiltern, deren begrenzte Transmission (Durchlässigkeit) für Anregungs- und Emissionslicht von Farbstoffen eine selektive Erfassung von Farbstofffluoreszenzen ermöglicht (Schmalbandfilter sind Bandpaßfilter, die nur für einen engen Spektralbereich durchlässig sind). Mit besonderem Vorteil kann dadurch vermieden werden, dass Lumineszenzen verschiedener Farbstoffe interferieren und insbesondere schwache Lumineszenzen von starken Lumineszenzen überlagert werden. Dadurch wird eine im Vergleich zu den aus dem Stand der Technik bekannten ELISPOT-Verfahren deutlich erhöhte Sensitivität für Immunzellen erreicht, die in Gegenwart eines Antigens zwei oder mehrere verschiedene Zytokine ausscheiden.

In einer besonders bevorzugten Ausführungsform werden pro Filtersatz Schmalbandfilter verwendet, die jeweils komplementär zu den spektralen Eigenschaften eines der Farbstoffe sind. Dadurch können in vorteilhafter Weise die bereits erwähnten Lumineszenzüberlagerungen vermieden werden. Die Filtersätze können neben Schmalbandfiltern auch Breitbandfilter aufweisen. Bevorzugt werden jedoch zur Visualisierung der immunkompetenten Zellen Filtersätze verwendet, die ausschließlich Schmalbandfilter aufweisen. Erfindungsgemäß ist es vorgesehen, dass jeder Filtersatz aus zwei Schmalbandfiltern besteht, wie in den Ansprüchen definiert. Pro Filtersatz wird in der Regel mit verschiedenen Schmalbandfiltertypen gearbeitet. Vorzugsweise wird pro Filtersatz ein Schmalband-Anregungsfilter und ein Schmalband-Sperrfilter verwendet. Der Schmalband-Anregungsfilter besitzt zweckmäßigerweise eine möglichst hohe Durchlässigkeit (Transmission) für Licht, welches zur Lumineszenzanregung der Farbstoffe vorgesehen ist. Im Falle von Fluoreszenz wird typischerweise längerwelliges (energiearmes) Licht von diesem Schmalband-Filtertyp zurückgehalten und steht daher für eine Anregung der Farbstoffe nicht zur Verfügung. Der Schmalband-Sperrfilter weist dagegen normalerweise eine hohe Durchlässigkeit für das von den Farbstoffen emittierte Licht auf. Im Falle der Fluoreszenz wird daher das kurzwellige (energiereiche) Anregungslicht möglichst vollständig eliminiert.

In den Filtersätzen werden Schmalband-Anregungsfilter verwendet, die für im Wesentlichen nicht überlappende Wellenlängenbereiche des Lichts, insbesondere von kurzwelligem Licht, durchlässig sind.

Die in den Filtersätzen verwendbaren Schmalband-Sperrfilter sind für im Wesentlichen nicht überlappende Wellenlängenbereiche des Lichts, insbesondere von längerwelligem Licht, durchlässig.

Im Rahmen des erfindungsgemäßen Verfahrens werden werden Fluoreszenz-Farbstoffe verwendet. Entsprechend wird im Rahmen des erfindungsgemäßen Verfahrens die Fluoreszenz von Farbstoffen gemessen.

Als mögliche Fluoreszenz-Farbstoffe können im Rahmen des erfindungsgemäßen Verfahrens insbesondere Cumarinderivate, Rhodaminderivate, Auraminderivate, Cyanine (Cy), Phycoerythrin, Allophycocyanin, Fluoreszeinisothiocyanat (FITC), Texas Red, 4'-6-Diamidino-2-phenylindol (DAPI), Propidiumiodid, Sp. green, Sp. orange, L. Yellow, Green Fluorescent Protein (GFP), Cyan fluoreszierendes Protein (CFP), Gelb fluoreszierendes Protein (YFP, Yellow Fluoresent Protein) und/oder Enhanced Green Fluorescent Protein (EGFP) verwendet werden. Beispiele für geeignete Cyanine sind Cyanin 2, Cyanin 3, Cyanin 5.5 und/oder Cyanin 5. Ein Beispiel für ein geeignetes Rhodaminderivat ist Tetramethyl-Rhodamin-Isothiocyanat (TRITC).

Erfindungsgemäß können die folgenden Farbstoffkombinationen zum Einsatz kommen: FITC/Texas Red, DAPI/FITC, DAPI/GFP, DAPI/Cy3, DAPI/TRITC, DAPI/Texas Red, DAPI/Cy5, FITC/TRITC, Sp. green/Sp. orange, Cy2/Cy3, L. Yellow/Rhodamin, FITC/Cy3, FITC/Propidiumiodid, FITC/Cy5 und/oder Cy3/Cy5.

Bevorzugt werden im Rahmen des erfindungsgemäßen Verfahrens Fluoreszenz-Filtersätze verwendet. Bei den verwendbaren Fluoreszenzfiltern kann es sich um Farbgläser (Absorptionsfiltern), Interferenzfilter oder Kombinationen davon handeln. Bevorzugt kommen Interferenzfilter zum Einsatz.

Gemäß einer weiteren bevorzugten Ausführungsform werden zur Visualisierung der immunkompetenten Zellen zumindest ein Filtersatz mit einem Schmalbandfilter für Fluoreszeinisothiocyanat und zumindest ein Filtersatz mit einem Schmalbandfilter für Cyanin 3 verwendet. Der Schmalbandfilter, der für die Anregung von Fluoreszeinisothiocyanat vorgesehen ist, weist vorzugsweise eine Durchlässigkeit für Licht im Wellenlängenbereich zwischen 460 und 500 nm auf. Der Schmalbandfilter, der für die Emission von Fluoreszeinisothiocyanat vorgesehen ist, weist bevorzugt eine Durchlässigkeit für Licht im Wellenlängenbereich zwischen 512 und 542 nm auf. Der bevorzugte Lichtdurchlässigkeitsbereich für einen Schmalbandfilter, der für die Anregung von Cyanin 3 vorgesehen ist, liegt im Wellenlängenbereich zwischen 541 und 551 nm. Der Lichtdurchlässigkeitsbereich für einen Schmalbandfilter, der für die Emission von Cyanin 3 vorgesehen ist, liegt vorzugsweise in einem Wellenlängenbereich zwischen 572 und 647 nm.

Im Rahmen des erfindungsgemäßen Verfahrens werden gewöhnlich Filtersätze verwendet, die neben den Schmalbandfiltern einen sogenannten Strahlteiler aufweisen. Der Strahlteiler sorgt mit besonderem Vorteil für eine optimale Lichtführung. So wird im Falle von Fluoreszenz kurzwelliges Licht reflektiert, wohingegen langwelliges Licht den Strahlteiler nahezu ungehindert passieren kann. Dadurch wird einerseits das Anregungslicht in Richtung der Farbstoffe "umgelenkt". Andererseits wirkt der Strahlteiler in besonders vorteilhafter Weise als zusätzliche Sperre für kurzwelliges Licht, welches ansonsten die Visualisierung von immunkompetenten Zellen stören würde. Erfindungsgemäß ist es insbesondere vorgesehen, dass die im Rahmen des erfindungsgemäßen Verfahrens verwendbaren Schmalbandfilter, insbesondere ein Schmalband-Anregungsfilter und ein Schmalband-Sperrfilter, zusammen mit einem Strahlteiler eine funktionelle Einheit bilden und vorzugsweise mechanisch in einem einzigen Element, einem sogenannten Filterblock, integriert werden. Das erfindungsgemäße Verfahren kann daher insbesondere mit zumindest zwei Filterblöcken der vorstehend beschriebenen Art durchgeführt werden.

Weiterhin können die Filtersätze auf einem vorzugsweise drehbaren Filterrad angeordnet sein. Die Drehung des Filterrads kann beispielsweise mit einem Schrittmotor erreicht werden.

Wie bereits erwähnt, besteht ein wesentlicher Vorteil des erfindungsgemäßen Verfahrens darin, dass je nach Schmalbandfilter spezifisch die Lumineszenz eines Farbstoffes mit seinen spektralen Eigenschaften, insbesondere Absorptions- und Emissionsmaxima, erfasst werden kann, ohne dass es zu einer störenden Überlagerung mit einer Lumineszenz eines anderen Farbstoffes kommt. Entsprechend ist es bevorzugt, dass zur Visualisierung der immunkompetenten Zellen die Lumineszenzen der Farbstoffe getrennt voneinander gemessen werden. Bevorzugt werden hierzu pro Testansatz, insbesondere pro Kavität oder Loch bzw. Vertiefung einer Mikrotiterplatte, zur Visualisierung der immunkompetenten Zellen zumindest zwei zweidimensionale Bilder erzeugt werden. Die zweidimensionalen Bilder werden gewöhnlich dadurch erzeugt, dass bei der Visualisierung der immunkompetenten Zellen unmittelbar erzeugte Farbbilder optisch vergrößert und in Bildpunkte zerlegt werden, die von einem Lesegerät getrennt erfasst und ausgewertet werden. Die Bildpunkte können als Zeilen abgelesen werden. Ein Computer kann dann aus den erhaltenen linearen Werten ein zweidimensionales Bild zusammensetzen. Im Rahmen des erfindungsgemäßen Verfahrens werden zur Messung der immunkompetenten Zellen vorzugsweise zumindest zwei bei der Visualisierung erzeugte zweidimensionale Bilder übereinander gelegt. Durch das Übereinanderlegen der Bilder, welches gewöhnlich mit Hilfe einer hierfür geeigneten Software erfolgt, können die zu messenden immunkompetenten Zellen identifiziert und insbesondere von anderen Zellen unterschieden werden.

Grundsätzlich kann zur Visualisierung der immunkompetenten Zellen die Anzahl von auf einer Untersuchungsfläche bzw. einem Träger auftretenden Spots bestimmt werden. Alternativ oder in Kombination dazu wird bevorzugt die Farbintensität einzelner Spots oder überlappender Spots, gegebenenfalls auch von Färbungen, die eine Untersuchungsfläche bedecken, quantitativ gemessen. Besonders bevorzugt wird eine Untersuchungsfläche in eine Vielzahl von Einzelpunkten zerlegt, die Farbintensität jedes Einzelpunktes getrennt gemessen und die gemessenen Intensitätswerte addiert. Erfindungsgemäß können bis 2 Millionen, insbesondere ca. 1,5 Millionen, Bildpunkte (Pixel) pro Untersuchungsfläche erfasst werden. Die Erfassung der Bildpunkte kann beispielsweise mit Hilfe einer Kamera erfolgen. Für die Messung der Farbintensität eines Einzelpunktes auf einer Untersuchungsfläche stehen mit besonderem Vorteil zwischen 200 und 300, insbesondere zwischen 220 und 260, vorzugsweise ca. 256 Abstufungen (Grauwerte), zur Verfügung. Die Verarbeitung und Auswertung der Bildpunkte kann beispielsweise mit einem Lesegerät vorgenommen werden. Vorzugsweise wird die Gesamtzahl der Bildpunkte sowie insbesondere ihre Intensität mit Hilfe eines sogenannten Image-Analyzers gemessen. Dadurch ist eine Messung der gesamten Färbung, bezogen auf eine Untersuchungsfläche oder einen bestimmten Teil davon, möglich. Als Referenzwert kann eine ungefärbte Stelle auf der einen Untersuchungsfläche, gegebenenfalls auch auf einer anderen Untersuchungsfläche, dienen. Als Maß für die Gesamtaktivität, d.h. die Gesamtintensität der Reaktionen aller Zellen auf einer Untersuchungsfläche auf ein definiertes Antigen, kann das Produkt aus der Anzahl der angeregten (gefärbten) Pixel und dem Farbwert (beispielsweise Graustufe zwischen 0 und 256) jedes angeregten Pixels herangezogen werden. Dieses Produkt wird zweckmäßigerweise durch 1000 geteilt, um einfach handhabbare Zahlenwerte (Einheiten) zu erhalten. Die Erfassung und Auswertung der Bildpunkte erfolgt vorzugsweise von oben, d.h. oberhalb einer Untersuchungsfläche. Die Verarbeitung zu einem zweidimensionalen Bild erfolgt, wie bereits erwähnt, gewöhnlich mit Hilfe von Computertechniken.

Gewöhnlich werden für den Inkubationsschritt eukaryotische Zellen humanen oder tierischen Ursprungs verwendet. Bevorzugt kommen humane Zellen zum Einsatz. Die eukaryotischen Zellen stammen in der Regel aus einem biologischen Untersuchungsmaterial. Bei dem Untersuchungsmaterial kann es sich um Körperflüssigkeiten, beispielsweise Blut, Lymphe, Gelenkflüssigkeit und/oder Schleimabsonderungen handeln. Bei dem Untersuchungsmaterial kann es sich zum Beispiel um eine Blutprobe, einen Cervix-Abstrich oder um eine Bronchiallavage handeln. Blutproben werden bevorzugt verwendet. Entsprechend ist es erfindungsgemäß bevorzugt, dass Blutzellen als eukaryotische Zellen verwendet werden. Bei den Blutzellen kann es sich insbesondere um B-Lymphozyten, T-Lymphozyten, Granulozyten, dendritische Zellen, Makrophagen und/oder Erythrozyten handeln. Unter immunkompetenten Zellen sollen im Sinne der Erfindung Immunzellen, insbesondere Blutzellen wie sie in diesem Abschnitt aufgeführt sind, verstanden werden.

Vorzugsweise werden die eukaryotischen Zellen vor der Inkubation mit dem Antigen angereichert, insbesondere von Erythrozyten befreit. Eine Anreicherung der eukaryotischen Zellen kann mit Hilfe konventioneller zellulärer Anreicherungstechniken durchgeführt werden. Beispielsweise können die eukaryotischen Zellen durch Zentrifugation, insbesondere Gradienten-Zentrifugation, angereichert werden. Für die Gradienten-Zentrifugation können beispielsweise Zucker-Gradienten verwendet werden. In einer weitergehenden Ausführungsform werden die eukaryotischen Zellen, vorzugsweise vor der Inkubation mit dem Antigen, von Blutserum, insbesondere autologem Blutserum, befreit. Dies geschieht gewöhnlich ausgehend von einer gewonnenen Blutprobe durch Abtrennung des flüssigen Blutanteils von zellulären Bestandteilen. Bezüglich der zellulären Bestandteile und geeigneter Abtrennungstechniken wird auf die in diesem Absatz gemachten Angaben Bezug genommen.

Das erfindungsgemäße Verfahren wird gewöhnlich auf einer hierfür geeigneten Untersuchungsfläche bzw. auf einem geeigneten Träger, insbesondere in einem Untersuchungsgefäß, durchgeführt. Bei der Untersuchungsfläche bzw. dem Träger kann es sich beispielsweise um Loch- oder Vertiefungsböden (Wells) von sogenannten Loch- oder Mikrotiterplatten handeln. Derartige Loch- bzw. Mikrotiterplatten, welche erfindungsgemäß bevorzugt als Untersuchungsgefäße zum Einsatz kommen, sind kommerziell erhältlich, insbesondere mit unterschiedlichen Loch- bzw. Vertiefungszahlen. So kann das erfindungsgemäße Verfahren beispielsweise mit Hilfe einer 96-Lochplatte durchgeführt werden. Pro Loch der Mikrotiterplatte können bis zu einer Million Zellen eingesetzt werden. Im Blut sind eine Million Zellen gewöhnlich in einem Milliliter Blut enthalten, so dass im Falle einer 96-Lochplatte ca. 96 ml Blut eingesetzt werden können. Die Mikrotiterplatten können weiterhin einen Vertiefungsdurchmesser von ca. 5 mm aufweisen, was einer Grundfläche von etwa 20 mm² entspricht. Die verwendete Untersuchungsfläche ist zweckmäßigerweise eben. Dadurch können lokale Konzentrationsunterschiede bei der Durchführung des erfindungsgemäßen Verfahrens vermieden werden. Außerdem eignen sich ebene Untersuchungsflächen grundsätzlich besser für die Erzeugung von zellulären Einfachschichten (Monolayer). Typische Materialien, aus denen geeignete Untersuchungsgefäße bestehen, sind beispielsweise Polystyrol, Polyvinylidendifluorid (PVDF), Nitrocellulose und Nylon.

In einer bevorzugten Ausführungsform werden zur Messung der immunkompetenten Zellen zumindest zwei verschiedene Abfangmoleküle und zumindest zwei verschiedene Detektionsmoleküle eingesetzt. Die Abfang- und Detektionsmoleküle sind vorzugsweise jeweils spezifisch für eines der ausgeschiedenen Zytokine. Typischerweise wird pro ausgeschiedenes Zytokin ein Abfangmolekül und ein entsprechendes Detektionsmolekül verwendet. Die Abfang- und Detektionsmoleküle binden gewöhnlich an verschiedenen Stellen der ausgeschiedenen Zytokine. Dadurch werden in der Regel ternäre Komplexstrukturen aus Abfangmolekülen, ausgeschiedenen Zytokinen und Detektionsmolekülen gebildet. Als Abfang- und Detektionsmoleküle werden in der Regel Antikörper, sogenannte Abfang-Antikörper und Detektions-Antikörper, eingesetzt. Bei den Abfang- und/oder Detektionsmolekülen kann es sich insbesondere um mono- und/oder polyklonale Antikörper handeln. Monoklonale Antikörper sind bevorzugt. Vorzugsweise sind die im Rahmen des erfindungsgemäßen Verfahrens einsetzbaren Detektionsmoleküle jeweils mit einem der Farbstoffe markiert. Die Markierung beruht meistens auf kovalenten Bindungen. Gewöhnlich bilden die Detektionsmoleküle eine Konjugatverbindung mit den Farbstoffen, wobei die Detektionsmoleküle und die Farbstoffe vorzugsweise kovalent miteinander verbunden sind.

Es wird die Anzahl an immunkompetenten Zellen gemessen, die zumindest ein Interferon und zumindest ein Interleukin ausscheiden.

Vorzugsweise werden im Rahmen des erfindungsgemäßen Verfahrens immunkompetente Zellen gemessen, die sowohl Interferon-γ als auch Interleukin-2 ausscheiden. Bevorzugt handelt es sich bei diesen Zellen um T-Zellen (T-Lymphozyten), insbesondere T_{H1}-Zellen, vorzugsweise davon abgeleitete Gedächtniszellen. Überraschenderweise konnte nämlich festgestellt werden, dass dieser Zelltyp während den verschiedenen Phasen einer Infektion in unterschiedlichen Mengen in Körperflüssigkeiten, insbesondere Blut, anwesend ist. So konnten große Mengen dieses Zelltyps vor allem in Blutproben von Testpersonen ermittelt werden, die entweder an einer latenten Infektion litten oder aber bei denen bereits eine Infektion über einen längeren Zeitraum abgeklungen war, so dass die Infektion im Wesentlichen als überstanden angesehen werden konnte. Typischerweise wiesen die Blutproben dieser Personen einen Anteil zwischen 30 und 50 % oder mehr des besagten Zelltyps auf, bezogen auf die in der Probe enthaltene Gesamtzahl an Zellen. Dagegen wiesen Blutproben von Personen, die an einer akuten Infektion litten, einen deutlich geringeren Anteil des fraglichen Zelltyps auf. In der Regel wiesen mit Hilfe des erfindungsgemäßen Verfahrens untersuchte Blutproben von Testpersonen, die an einer akuten Infektion litten, einen Anteil des fraglichen Zelltyps zwischen 10 und 20 % auf, bezogen auf die in der Blutprobe enthaltene Gesamtzahl an Zellen. Somit eignet sich die Messung von immunkompetenten Zellen, welche sowohl Interferon-γ als auch Interleukin-2 ausscheiden, in besonders vorteilhafter Weise für eine Beurteilung des Immunstatus, insbesondere zur Unterscheidung zwischen akuten Infektionen einerseits und latenten oder überstandenen Infektionen andererseits. Zusätzlich eignet sich die Messung von immunkompetenten Zellen, welche Interferon-γ und Interleukin-2 ausscheiden, zur Impfkontrolle. Können viele Zellen dieses Zelltyps nachgewiesen werden, bedeutet dies einen guten Impfstatus.

Bevorzugt werden Fragmente von Erregertypen, insbesondere Erregerepitope als Antigene verwendet. Bei den Epitopen handelt es sich insbesondere um Peptide, vorzugsweise Oligopeptide. Geeignete Epitope können aus 5 bis 25, insbesondere 9 bis 11, Aminosäureeinheiten aufgebaut sein. In einer bevorzugten Ausführungsform wird zur Inkubation der eukaryotischen Zellen zumindest ein Antigen aus der Gruppe PPD, RD 1, RD 2, ESAT 6, CFP 10, MPT 41, MTB 64, PPE 44, OSP A, OSP B, OSP C, OSP D, OSP E, VIsE, p 58, p 100 und Dbp A verwendet.

Zur Inkubation der eukaryotischen Zellen wird zumindest ein Tuberkulose spezifisches Antigen, insbesondere aus der Gruppe PPD, RD 1, RD 2, MPT 64, MTB 41 und PPE 44, eingesetzt. Zur Inkubation der eukaryotischen Zellen wird zumindest ein Borreliose spezifisches Antigen, insbesondere aus der Gruppe VIsE, OSP A, OSP B, OSP C, OSP D und OSP E, verwendet.

Bei den Infektionen bzw. Infektionserkrankungen, welche mit Hilfe des erfindungsgemäßen Verfahrens in vitro diagnostiziert bzw. deren Therapieverlauf in vitro überwacht werden kann, handelt es sich um Tuberkulose oder Borreliose.
Das erfindungsgemäße Verfahren eignet sich besonders bevorzugt zur Beurteilung des Immunstatus, insbesondere zur Unterscheidung zwischen akuten bzw. aktiven Infektionen einerseits und latenten bzw. chronischen oder überstandenen Infektionen andererseits. Wie bereits erwähnt, konnte im Rahmen der Erfindung gezeigt werden, dass akute Infektionen durch eine geringe Anzahl von immunkompetenten Zellen charakterisiert sind, welche sowohl Interferon-γ als auch Interleukin-2 ausscheiden (sezernieren), insbesondere im Verhältnis zu Zellen, die nur Interferon-γ ausscheiden. Bei latenten Infektionen liegen die Verhältnisse dagegen gerade umgekehrt.

Je nach Infektion bzw. Infektionserkrankung sind unterschiedliche Bezeichnungen für den Infektionsstatus gebräuchlich. So spricht man im Falle der Tuberkulose von akuten und latenten Infektionen bzw. Infektionserkrankungen. Akute Tuberkulose stellt ein aktives Stadium der Erkrankung dar. Patienten, die an einer akuten Tuberkulose leiden, produzieren Tuberkulose-Erreger und sind infektiös (kontagiös). Latente Tuberkulose-Infektionen sind in diesem Kontext nicht infektiös. Es sind gewöhnlich zumindest direkt keine Tuberkulose-Erreger nachweisbar. Bei der Borreliose werden dagegen normalerweise die Bezeichnungen aktive und chronische Infektionen bzw. Infektionserkrankungen verwendet. Erfindungsgemäß ist es besonders bevorzugt, dass die Überprüfung des Immunstatus durch Messung der bereits erwähnten immunkompetenten Zellen vorgenommen wird, welche sowohl Interferon-γ als auch Interleukin-2 in Gegenwart eines Antigens ausscheiden. Weiterhin eignet sich das Verfahren auch zur Impfprognose oder zur Beurteilung des Impfstatus. Mit Hilfe des erfindungsgemäßen Verfahrens kann daher mit besonderem Vorteil überprüft werden, ob ein ausreichender Impfschutz besteht oder gegebenenfalls eine Nachimpfung erforderlich ist. Bezüglich weiterer Merkmale und Einzelheiten wird daher auf die bisherige Beschreibung verwiesen.

Weitere Einzelheiten und Merkmale der Erfindung ergeben sich aus der nachfolgenden Beschreibung der Darstellung des erfindungsgemäßen Verfahrens anhand von zwei Beispielen in Verbindung mit den Figuren und Unteransprüchen. Hierbei können die jeweiligen Merkmale für sich alleine oder zu mehreren in Kombination miteinander verwirklicht sein. Die Figuren werden hiermit durch ausdrückliche Bezugnahme zum Inhalt dieser Beschreibung gemacht.

In den Figuren zeigen schematisch:
Figur 1: Die spektralen Eigenschaften eines für die Detektion der Farbstoffe Cyanin 3, TRITC und Alexa 546 geeigneten Sets aus Schmalbandfiltern (Bright Line HC 593/40, HC-Strahlteiler BS 562, Bright Line HC 543/22),
Figur 2: Die spektralen Eigenschaften eines für die Detektion der Farbstoffe FITC, EGFP, Cyanin 2 und Alexa 488 geeigneten Sets aus Schmalbandfiltern (Bright Line HC 520/35, HC-Strahlteiler BS 495 und Bright Line HC 472/30),
Figur 3: Die spektralen Eigenschaften eines für die Detektion des Farbstoffs DAPI geeigneten Sets aus Schmalbandfiltern (Bright Line HC 447/60, HC-Strahlteiler BS 409, Bright Line HC 387/11),
Figur 4: Die spektralen Eigenschaften eines für die Detektion der Farbstoffe Cyanin 5 und Alexa 647 geeigneten Sets aus Schmalbandfiltern (Bright Line HC 692/40, HC-Strahlteiler BS 660, Bright Line HC 628/40),
Figur 5: Die spektralen Eigenschaften eines für die Detektion des Farbstoffs CFP geeigneten Sets aus Schmalbandfiltern (Bright Line HC 483/32, HC-Strahlteiler BS 458, Bright Line HC 438/24),
Figur 6: Die spektralen Eigenschaften eines für die Detektion des Farbstoffs YFP geeigneten Sets aus Schmalbandfiltern (Bright Line HC 542/27, HC-Strahlteiler BS 520, Bright Line HC 500/24),
Figur 7: Die spektralen Eigenschaften eines für die Detektion der Farbstoffe Texas Red und mCherry geeigneten Sets aus Schmallbandfiltern (Bright Line HC 624/40, HC-Strahlteiler BS 593, Bright Line HC 562/40),
Figur 8: Die spektralen Eigenschaften eines für die Detektion des Farbstoffs Cy5.5 geeigneten Sets aus Schmalbandfiltern (Bright Line HC 716/40, HC-Strahlteiler BS 685, Bright Line HC 655/40),

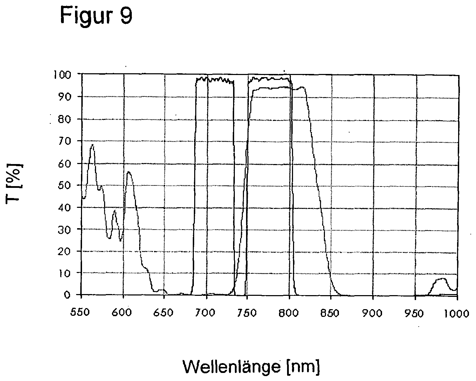

Figur 9: Die spektralen Eigenschaften eines für die Detektion des Farbstoffs Cy7 geeigneten Sets aus Schmalbandfiltern (Bright Line HC 775/46, HC-Strahlteiler BS 741, Bright Line HC 710/40).

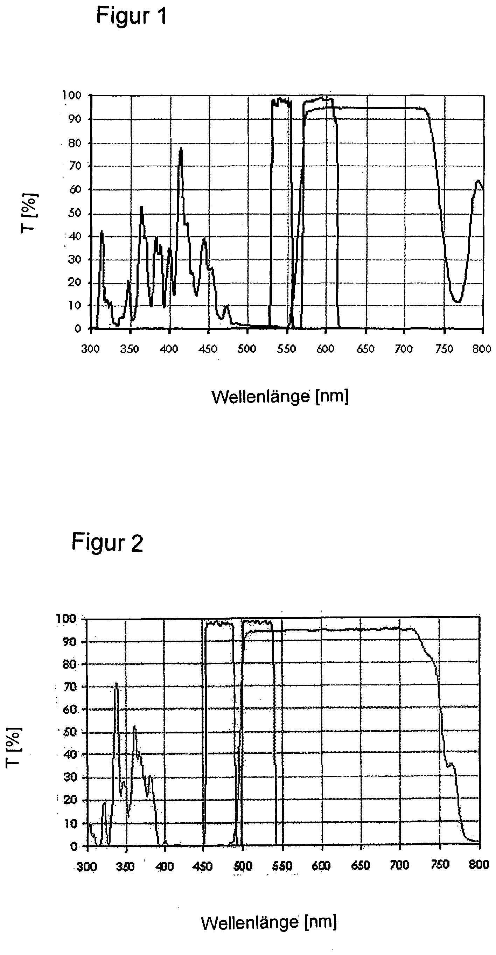

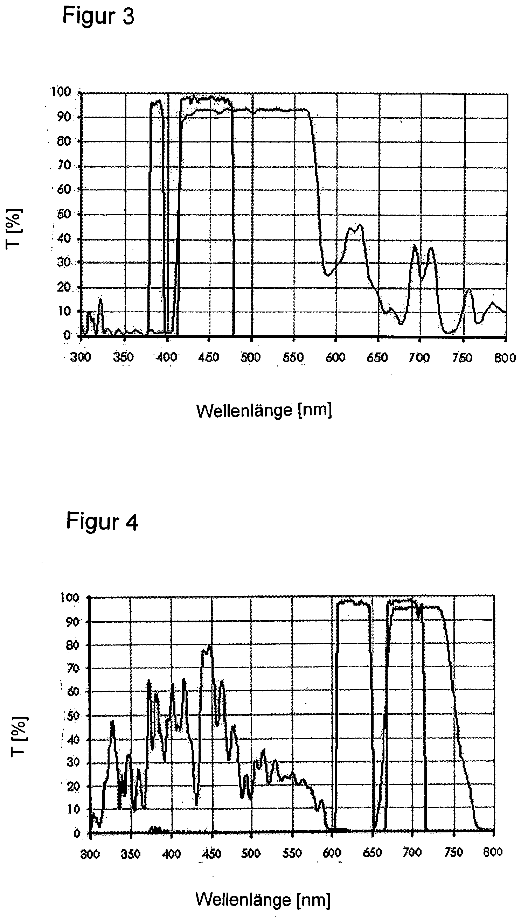

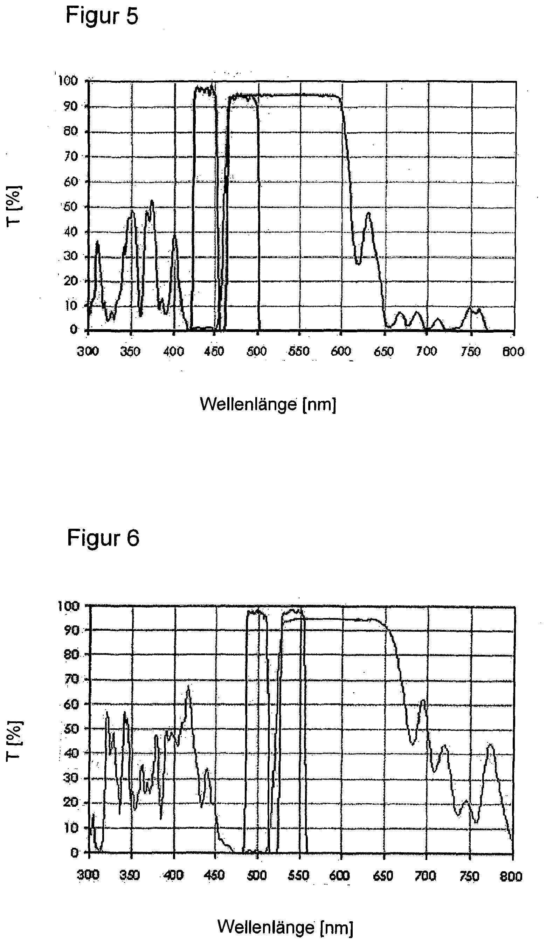

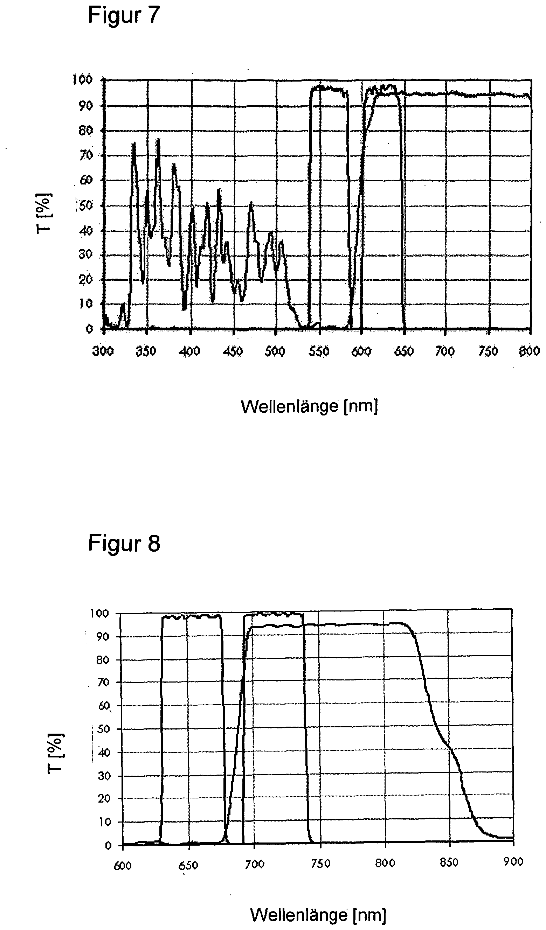

In den Figuren 1 bis 8 ist auf der Ordinate jeweils die Transmission in Prozent und auf der Ordinate die Wellenlänge in Nanometer aufgetragen.

### Beispiel 1:

### Nachweis von Tuberkulose-Erreger

Zur Durchführung des Verfahrens wird eine 96-Lochplatte verwendet. Zunächst werden zwei verschiedene Anti-Antikörper als Abfangmoleküle auf den Böden der Vertiefungen der Lochplatte immobilisiert. Der eine Anti-Antikörper ist spezifisch gegen Interferon-γ, der andere Anti-Antikörper spezifisch gegen Interleukin-2 gerichtet. Danach wird PPD als Tuberkulose-Erreger auf dem Boden der Vertiefungen immobilisiert. Zusätzlich oder alternativ dazu können auch Peptide vom sogenannten RD1- und RD2-Komplex benutzt werden. Des Weiteren können auch weitere Proteine von Mycobacterium Tuberculosum (38 kD, 41 kD, 44 kD, 64 kD) eingesetzt werden. Anschließend werden pro Vertiefung 100.000 bis 250.000 PBMCs (peripheral blood mononuclear cells) hinzugegeben. Die Inkubation der Blutzellen (zusammen mit den Tuberkulose-Erregern) wird während eines Zeitraums von ca. 18 Stunden bei ca. 37 °C vorgenommen. Danach werden die Zellen dekantiert und die Lochplatte mehrfach gewaschen. Anschließend wird ein gegen Interferon-γ gerichteter Antikörper, der mit Fluoreszeinisothiocyanat kovalent markiert ist, und ein gegen Interleukin-2 gerichteter Antikörper, der mit Cy3 kovalent markiert ist, hinzugegeben. Es wird eine erneute Inkubation bei ca. 37 °C während 4 bis 12 Stunden vorgenommen. Anschließend werden nicht gebundene Farbstoff markierte Antikörper von dem Lochplattenboden der 96-Lochplatte abgespült. Es werden dann die Fluoreszenzen der Farbstoffe gemessen. Zu diesem Zweck werden pro Loch der Lochplatte zwei Bilder erzeugt, eines im grünen Spektralbereich (grüne Fluoreszenz von Fluoreszeinisothiocyanat) und eines im roten Spektralbereich (rote Fluoreszenz von Cy3). Dadurch wird gewährleistet, dass weder schwache grüne noch schwache rote Fluoreszenzen durch entsprechende starke rote bzw. starke grüne Fluoreszenzen überlagert werden. Anschließend werden die Bilder übereinander gelegt. Dadurch können immunkompetente Zellen identifiziert und insbesondere quantifiziert werden, die sowohl Interferon-γ als auch Interleukin-2 ausscheiden. Beträgt der Anteil der Zellen, die sowohl Interferon-γ als auch Interleukin-2 ausscheiden, weniger als 30%, bezogen auf die Gesamtzahl der insgesamt untersuchten Zellen, stammen die Zellen von Patienten, die an einer akuten Tuberkulose leiden. Liegt der Anteil dieses Zelltyps über 30%, insbesondere über 30 bis 50% oder mehr, so stammen die Zellen insgesamt von Patienten, die entweder an einer latenten Tuberkulose-Infektion leiden oder von Patienten mit einer bereits überstandenen Infektion.

### Beispiel 2:

### Nachweis von Borrelien

Zur Durchführung des Verfahrens wird eine 96-Lochplatte verwendet. Auf den Böden der Vertiefungen der Lochplatte werden zwei verschiedene Anti-Antikörper immobilisiert, die spezifisch gegen Interleukin-γ bzw. Interleukin-2 gerichtet sind. Anschließend werden Borrelien spezifische Antigene, beispielsweise die Peptide OSP A, OSP B, OSP C und/oder VIsE inneres Flaggelin Fragment benutzt. Die Borrelien spezifischen Antigene werden mit einer Konzentration von 1 bis 10 µg/ml auf den Böden der Vertiefungen gekoppelt. Danach werden pro Vertiefungen 100.000 bis 250.000 PBMCs (peripheral blood mononuclear cells) hinzugegeben. Die Blutzellen werden (zusammen mit den Borrelien spezifischen Antigenen) während eines Zeitraums von ca. 18 Stunden bei ca. 37 °C inkubiert. Danach werden die Blutzellen dekantiert und die Lochplatte mehrfach gewaschen. Anschließend wird ein gegen Interferon-γ gerichteter Antikörper, der mit Fluoreszeinisothiocyanat kovalent markiert ist, und ein gegen Interleukin-2 gerichteter Antikörper, der mit Cy3 kovalent markiert ist, hinzugegeben. Es wird eine erneute Inkubation bei ca. 37 °C während 4 bis 12 Stunden vorgenommen. Anschließend werden nicht gebundene Farbstoff markierte Antikörper von dem Lochplattenboden der 96-Lochplatte abgespült. Es werden dann die Fluoreszenzen der Farbstoffe, wie unter Beispiel 1 beschrieben, gemessen. Beträgt der Anteil der Zellen, die sowohl Interferon-γ als auch Interleukin-2 ausscheiden, weniger als 30%, bezogen auf die Gesamtzahl der insgesamt untersuchten Zellen, stammen die Zellen von Patienten, die an einer aktiven Borreliose leiden. Liegt der Anteil dieses Zelltyps über 30%, insbesondere über 30 bis 50% oder mehr, so stammen die Zellen insgesamt von Patienten, die entweder an einer chronischen Borreliose-Infektion leiden oder von Patienten mit einer bereits überstandenen Infektion.

## Patentansprüche

1. ELISPOT-Verfahren zur in vitro-Diagnose und/ oder in vitro-Therapieüberwachung von Tuberkulose oder Borreliose, **dadurch gekennzeichnet, dass** eukaryotische Zellen mit einem Tuberkulose spezifischen oder Borreliose spezifischen Antigen inkubiert werden und die Anzahl an immunkompetenten Zellen gemessen wird, die zumindest ein Interferon und zumindest ein Interleukin als Reaktion auf das Antigen ausscheiden, wobei zur Messung der immunkompetenten Zellen diese zumindest mit Hilfe von zwei verschiedenen Fluoreszenz-Farbstoffen und zumindest zwei verschiedenen Filtersätzen visualisiert werden, wobei die Filtersätze Schmalbandfilter aufweisen, wobei in den Filtersätzen Schmalband-Anregungsfilter, die für sich im Wesentlichen nicht überlappende Wellenlängenbereiche des Lichts durchlässig sind, und Schmalband-Sperrfilter, die für sich im Wesentlichen nicht überlappende Wellenlängenbereiche des Lichts durchlässig sind, verwendet werden.

2. Verfahren nach Anspruch 1, **dadurch gekennzeichnet, dass** pro Filtersatz Schmalbandfilter verwendet werden, die jeweils komplementär zu den spektralen Eigenschaften eines der Farbstoffe sind, wobei zur Visualisierung vorzugsweise Filtersätze verwendet werden, die ausschließlich Schmalbandfilter aufweisen.

3. Verfahren nach Anspruch 1 oder 2, **dadurch gekennzeichnet, dass** pro Filtersatz mit zwei Schmalbandfiltern gearbeitet wird, wobei vorzugsweise pro Filtersatz mit verschiedenen Schmalbandfiltertypen gearbeitet wird.

4. Verfahren nach einem der vorhergehenden Ansprüche, **dadurch gekennzeichnet, dass** pro Filtersatz ein Schmalband-Anregungsfilter und ein Schmalband-Sperrfilter verwendet wird.

5. Verfahren nach einem der vorhergehenden Ansprüche, **dadurch gekennzeichnet, dass** zur Visualisierung der immunkompetenten Zellen mit zwei Filtersätzen gearbeitet wird, insbesondere einem Filtersatz mit Schmalbandfilter für Fluoreszeinisothiocyanat und einem Filtersatz mit Schmalbandfilter für Cyanine 3.

6. Verfahren nach einem der vorhergehenden Ansprüche, **dadurch gekennzeichnet, dass** zur Messung der immunkompetenten Zellen zumindest zwei verschiedene Abfangmoleküle und zumindest zwei verschiedene Detektionsmoleküle eingesetzt werden, wobei vorzugsweise zur Messung der immunkompetenten Zellen zumindest zwei Detektionsmoleküle eingesetzt werden, die jeweils mit einem der Farbstoffe markiert sind.

7. Verfahren nach einem der vorhergehenden Ansprüche, **dadurch gekennzeichnet, dass** die Anzahl an immunkompetenten Zellen, insbesondere T-Zellen, vorzugsweise T_{H1}-Zellen, gemessen wird, die Interferon-γ und Interleukin-2 ausscheiden.

8. Verfahren nach einem der vorhergehenden Ansprüche, **dadurch gekennzeichnet, dass** zur Inkubation der eukaryotischen Zellen zumindest ein Tuberkulose spezifisches Antigen aus der Gruppe PPD, RD 1, RD 2, MPT 64, MTB 41 und PPE 44, verwendet wird.

9. Verfahren nach einem der vorhergehenden Ansprüche, **dadurch gekennzeichnet, dass** zur Inkubation der eukaryotischen Zellen zumindest ein Borreliose spezifisches Antigen aus der Gruppe VIsE, OSP A, OSP B, OSP C, OSP D und OSP E, eingesetzt wird.

10. Verwendung des Verfahrens nach einem der vorhergehenden Ansprüche zur Überprüfung des Infektionsstatus, insbesondere zur Unterscheidung zwischen akuten Infektionen einerseits und latenten oder überstandenen Infektionen andererseits.

11. Verwendung des Verfahrens nach einem der vorhergehenden Ansprüche 1 bis 9 zur Impfprognose.

## Claims

1. ELISPOT method for the in vitro diagnosis and/or in vitro therapy monitoring of tuberculosis or borreliosis, **characterized in that** eukaryotic cells are incubated with a tuberculosis-specific antigen or borreliosis-specific antigen and the number of immunocompetent cells which secrete at least one interferon and at least one interleukin as a reaction to the antigen is measured, wherein for measuring the immunocompetent cells, they are visualized with the aid of at least two different fluorescent dyes and at least two different filter sets, wherein the filter sets comprise narrowband filters, wherein in the filter sets are used narrowband excitation filters, that are transmissive for essentially non-overlapping wavelength ranges of light, and narrowband blocking filters, that are transmissive for essentially non-overlapping wavelength ranges of light.

2. Method according to claim 1, **characterized in that** narrowband filters which are respectively complementary to the spectral properties of one of the dyes are used for each filter set, wherein preferably filter sets are used for visualization which exclusively comprise narrowband filters.

3. Method according to claim 1 or 2, **characterized in that** operation is carried out with two narrowband filters per filter set, wherein preferably operation is carried out with different narrowband filter types in each filter set.

4. Method according to any of the preceding claims, **characterized in that** one narrowband excitation filter and one narrowband blocking filter are used for each filter set.

5. Method according to any of the preceding claims, **characterized in that** for visualization of the immunocompetent cells, operation is carried out with two filter sets, in particular one filter set having narrowband filters for fluorescein isothiocyanate and one filter set having narrowband filters for cyanine 3.

6. Method according to any of the preceding claims, **characterized in that** for measuring the immunocompetent cells, at least two different capture molecules and at least two different detection molecules are used, wherein preferably for measuring the immunocompetent cells, at least two detection molecules are used, each of which is labeled with one of the dyes.

7. Method according to any of the preceding claims, **characterized in that** the number of immunocompetent cells, in particular T cells, preferably T_{H1} cells, which secrete interferon-γ and interleukin-2, is measured.

8. Method according to any of the preceding claims, **characterized in that** for incubating the eukaryotic cells, at least one tuberculosis-specific antigen from the group consisting of PPD, RD 1, RD 2, MPT 64, MTB 41 and PPE 44, is used.

9. Method according to any of the preceding claims, **characterized in that** for incubating the eukaryotic cells, at least one borreliosis-specific antigen from the group consisting of VIsE, OSP A, OSP B, OSP C, OSP D and OSP E, is used.

10. Use of the method according to any of the preceding claims for testing the infection status, in particular for distinguishing between acute infections on the one hand and latent or recovered infections on the other hand.

11. Use of the method according to any of the preceding claims 1 to 9 for immunization prognosis.

## Revendications

1. Procédé ELISPOT pour le diagnostic in vitro et/ou le suivi de traitement in vitro de la tuberculose ou de la borréliose, **caractérisé en ce que** des cellules eucaryotes sont incubées avec un antigène spécifique à la tuberculose ou spécifique à la borréliose, et le nombre de cellules immunocompétentes, qui excrètent au moins un interféron et au moins une interleukine en tant que réaction à l'antigène est mesuré, les cellules immunocompétentes étant visualisées pour leur mesure au moins à l'aide de deux colorants fluorescents différents et d'au moins deux groupes de filtres différents, les groupes de filtres comprenant des filtres à bande étroite, des filtres d'excitation à bande étroite, qui sont perméables à des plages de longueurs d'onde essentiellement non chevauchantes de la lumière, et des filtres de coupure à bande étroite, qui sont perméables à des plages de longueurs d'onde essentiellement non chevauchantes de la lumière, étant utilisés dans les groupes de filtres.

2. Procédé selon la revendication 1, **caractérisé en ce que** des filtres à bande étroite qui sont chacun complémentaires des propriétés spectrales d'un des colorants sont utilisés dans chaque groupe de filtres, des groupes de filtres qui comprennent exclusivement des filtres à bande étroite étant utilisés de préférence pour la visualisation.

3. Procédé selon la revendication 1 ou 2, **caractérisé en ce que** deux filtres à bande étroite sont utilisés par groupe de filtre, différents types de filtres à bande étroite étant de préférence utilisés par groupe de filtre.

4. Procédé selon l'une quelconque des revendications précédentes, **caractérisé en ce qu'**un filtre d'excitation à bande étroite et un filtre de coupure à bande étroite sont utilisés par groupe de filtres.

5. Procédé selon l'une quelconque des revendications précédentes, **caractérisé en ce que** deux groupes de filtres sont utilisés pour la visualisation des cellules immunocompétentes, notamment un groupe de filtres contenant un filtre à bande étroite pour l'isothiocyanate de fluorescéine et un groupe de filtre contenant un filtre à bande étroite pour la cyanine 3.

6. Procédé selon l'une quelconque des revendications précédentes, **caractérisé en ce qu'**au moins deux molécules de piégeage différentes et au moins deux molécules de détection différentes sont utilisées pour la mesure des cellules immunocompétentes, au moins deux molécules de détection qui sont chacune marquées avec un des colorants étant de préférence utilisées pour la mesure des cellules immunocompétentes.

7. Procédé selon l'une quelconque des revendications précédentes, **caractérisé en ce que** le nombre de cellules immunocompétentes, notamment de cellules T, de préférence de cellules T_{H1}, qui excrètent l'interféron γ et l'interleukine 2 est mesuré.

8. Procédé selon l'une quelconque des revendications précédentes, **caractérisé en ce qu'**au moins un antigène spécifique à la tuberculose du groupe constitué par PPD, RD 1, RD 2, MPT 64, MTB 41 et PPE 44 est utilisé pour l'incubation des cellules eucaryotes.

9. Procédé selon l'une quelconque des revendications précédentes, **caractérisé en ce qu'**au moins un antigène spécifique à la borréliose du groupe constitué par VIsE, OSP A, OSP B, OSP C, OSP D et OSP E est utilisé pour l'incubation des cellules eucaryotes.

10. Utilisation du procédé selon l'une quelconque des revendications précédentes pour vérifier l'état d'infection, notamment pour différencier des infections aigües d'une part et des infections latentes ou surmontées d'autre part.

11. Utilisation du procédé selon l'une quelconque des revendications 1 à 9 précédentes pour le pronostic de vaccinations.
